# EUROPÄISCHE PATENTANMELDUNG

(11) **EP 0 627 224 A1**
(43) Veröffentlichungstag der Anmeldung: **07.12.1994**
(21) Anmeldenummer: 94108451.9
(22) Anmeldetag: 01.06.1994
(51) Int. Cl.: A61L 9/03

(54) **Vorrichtung zur Abgabe von Duftstoffen in einen Raum**

(30) Priorität: 04.06.1993 DE 9308368 U
(71) Anmelder: VOITINO DESIGN ITALIA S.r.l., I-39043 Klausen (IT)
(72) Erfinder: Voit, Hans, D-82166 Gräfelfing (DE)
(74) Vertreter: Haft, von Puttkamer, Berngruber, Czybulka

(57) **Zusammenfassung**

Eine Vorrichtung zur Abgabe von Duftstoffen in einen Raum weist eine Dosiereinrichtung, vorzugsweise eine Schlauchpumpe, auf, mit der eine Lösung des Duftstoffs von einem Vorratsbehälter dem Verdampfungsgefäß zugeführt wird.

## Beschreibung

Die Erfindung bezieht sich auf eine Vorrichtung zur Abgabe von Duftstoffen in einen Raum durch Erwärmung des Duftstoffs in einem Verdampfungsgefäß mit einer elektrischen Heizeinrichtung.

Wenn bei den bekannten Vorrichtungen dieser Art der Behälter mit dem Duftstoff gefüllt ist, entsteht zunächst ein sehr starker, penetranter Duft. Wird hingegen der Behälter mit weniger Duftstoff versetzt, geht die Wirkung nach kurzer Zeit verloren.

Aufgabe der Erfindung ist es daher, Räume unterschiedlicher Größe über beliebig lange Zeit mit einer angenehmen Duftstoffkonzentration zu beduften.

Dies wird erfindungsgemäß mit der im Anspruch 1 gekennzeichneten Vorrichtung erreicht. In den Unteransprüchen sind vorteilhafte Ausgestaltungen der Erfindung wiedergegeben.

Mit der erfindungsgemäßen Vorrichtung wird der Boden des erwärmten Verdampfungsgefäßes mit einem dünnen Film der Duftstofflösung bedeckt. Damit wird eine gleichmäßige Beduftung sichergestellt.

Die Duftstofflösung wird dem Verdampfungsgefäß vorzugsweise von der Seite oder von oben zugeführt. Wenn eine als Schlauchpumpe ausgebildete Dosiereinrichtung verwendet wird, kann ein Zeitschalter vorgesehen sein, der die Schlauchpumpe in Intervallen betätigt. Damit gelangt die Duftstofflösung in Tropfen in das Verdampfungsgefäß, welche sich auf dem flachen Boden zu einem dünnen Film ausbreiten. Damit dieser Film eine vorgegebene Stärke nicht überschreitet, ist im Boden des Verdampfungsgefäßes eine Austrittsöffnung vorgesehen, von der die nicht verdampfte Duftstofflösung in den Vorratsbehälter zurückfließt.

Durch eine spiralförmige Nut im Boden des Verdampfungsgefäßes kann der Strömungsweg der Duftstofflösung bis zu dieser Austrittsöffnung verlängert werden.

Da bei der erfindungsgemäßen Vorrichtung der Verbrauch an Duftstofflösung sehr gering ist, können relativ kostspielige ätherische Öle als Duftstoffe verwendet werden, die vorzugsweise in alkoholischer Lösung eingesetzt werden. Ätherische Öle werden als wesentlich angenehmer empfunden als synthetische Duftstoffe.

Durch die Kreislaufführung der Lösung von dem Vorratsbehälter über die Dosiereinrichtung bzw. Schlauchpumpe zu dem Verdampfungsgefäß und vom Verdampfungsgefäß zum Vorratsbehälter zurück, ist die erfindungsgemäße Vorrichtung auch leicht zu reinigen. Dazu braucht die Duftstofflösung im Vorratsbehälter lediglich durch eine Reinigungsflüssigkeit, beispielsweise reinen Alkohol, ersetzt zu werden.

Die erfindungsgemäße Vorrichtung kann zur Beduftung von Räumen mit mehreren Hundert oder Tausend Kubikmeter ausgelegt sein. Um die gewünschte Duftstoffkonzentration in der Raumluft einzustellen, braucht lediglich das Zeitintervall vorgegeben zu werden, nach dem die Dosiereinrichtung bzw. die Schlauchpumpe die Duftstofflösung tropfenförmig in das Verdampfungsgefäß einspeist. Dazu kann eine Tabelle vorgesehen sein, auf der einerseits das Raumvolumen und andererseits das einzustellende Zeitintervall angegeben ist.

Die erfindungsgemäße Vorrichtung kann außerdem mit einer Zeitschaltuhr versehen sein, so daß sie den Raum z. B. nur zu einer bestimmten Tageszeit beduftet.

Wenn zu unterschiedlichen Zeiten unterschiedliche Düfte abgegeben werden sollen, also z. B. vormittags Limone, nachmittags Eukalyptus oder Lavendel, kann man auch mehrere derartige Vorrichtungen in dem betreffenden Raum aufstellen, ggf. zusammengefaßt zu einer Einheit.

Nachstehend ist eine Ausführungsform der erfindungsgemäßen Vorrichtung anhand der Zeichnung näher erläutert.

Darin zeigen:
- Fig. 1: eine Ansicht der Rückseite der Vorrichtung mit abgenommener Abdeckung und abgenommener Schalterbefestigungsplatte;
- Fig. 2: eine Ansicht des oberen Abschnitts der Vorrichtung von vorne; und
- Fig. 3: eine Draufsicht auf den Verdampfungsbehälter.

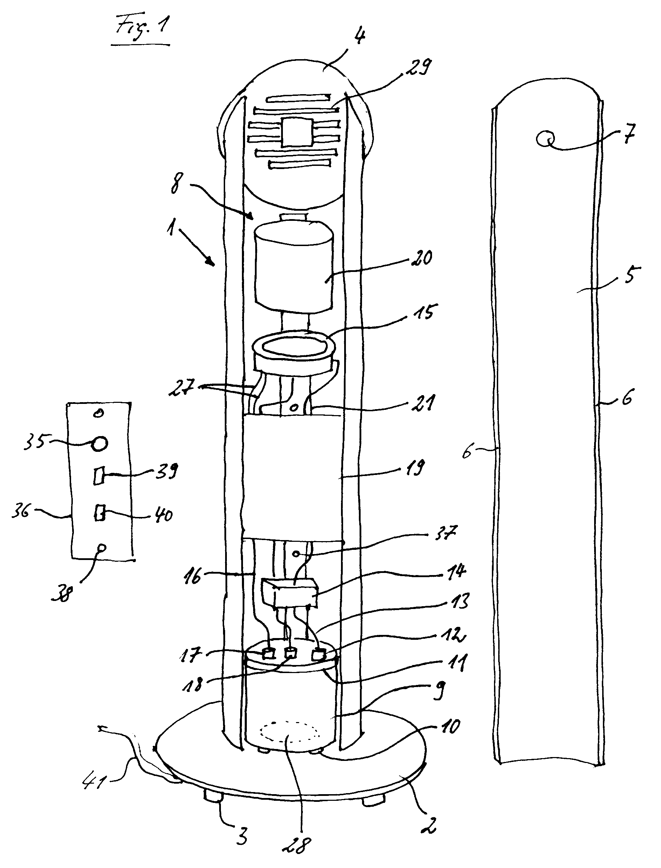

Gemäß Fig. 1 weist die Vorrichtung ein säulenförmiges Gehäuse 1 auf, das als Rohr ausgebildet ist. Das Gehäuse 1 ist auf einer scheibenförmigen Platte 2 senkrecht angeordnet, welche den Standfuß bildet. An der Unterseite der Platte 2 sind Füßchen 3 aus elastischem Material befestigt.

Am oberen Ende ist das säulenförmige Gehäuse 1 durch eine ovale Platte 4 verschlossen, welche sich von der Rückseite der Vorrichtung schräg nach unten erstreckt.

Die Rückseite des Gehäuses 1 ist mit einer Abdeckung 5 verschließbar, die als schalenförmiges, dem rohrförmigen Gehäuse 1 entsprechendes Segment ausgebildet ist.

Zur Befestigung der Abdeckung 5 an der Rückseite des Gehäuses 1 weist die Abdeckung 5 an ihren beiden Längsseiten jeweils nach innen und seitwärts ragende Randabschnitte 6 auf, die nicht dargestellte Nuten an der Innenseite an den Längskanten der Öffnung 8 übergreifen und damit eine Klemmverbindung zwischen der Abdeckung 5 und dem Gehäuse 1 herstellen. Um die Abdeckung 5 abzunehmen, weist sie ein Griffloch 7 auf. Freilich kann auch eine andere Ausbildung der Klemmverbindung oder eine andere Befestigung der Abdeckung 5 an dem Gehäuse 1 vorgesehen sein. Auch kann das Gehäuse 1 statt zylindrisch auch rechteckig oder in anderer Weise prismatisch ausgebildet sein.

Das Gehäuse 1, die Bodenplatte 2 und die Platte 4 an der Oberseite sowie die Abdeckung 5 können aus Metall, z. B. Leichtmetallspritzguß, hergestellt sein oder aus einem anderen Material bestehen, beispielsweise Kunststoff.

Das Gehäuse 1 ist etwa 1 bis 1,5 m hoch, so daß die schräge Platte 4 auf seiner Oberseite gut sichtbar ist.

In der Säule 1 ist unten ein Vorratsbehälter 9 für die Duftstofflösung vorgesehen. Der Vorratsbehälter 9 ist durch Füßchen 10 mit Abstand von der Bodenplatte 2 angeordnet.

Der Vorratsbehälter 9 ist mit einem Deckel 11 verschließbar, welcher einen Anschluß 12 für eine Leitung 13 aufweist, die über eine Schlauchpumpe 14 zu einem Verdampfungsgefäß 15 nach oben führt. Eine Rückflußleitung 16 führt von dem Verdampfungsgefäß 15 nach unten zu dem Vorratsbehälter 9, an dessen Deckel 11 sie mit einem Anschluß 17 befestigt ist. Ein elektrischer Anschluß 18 im Deckel 11 ist für eine nicht dargestellte Füllstandssonde im Behälter 9 vorgesehen.

Zwischen der Pumpe 14 und dem Verdampfungsgefäß 15 ist eine Platine 19 vorgesehen, die die nicht dargestellte elektrische Steuerschaltung der Vorrichtung aufweist.

Im Abstand oberhalb des Verdampfungsgefäßes 15 ist in einem rohrförmigen Ventilatorgehäuse 20 ein nicht dargestellter Ventilator angeordnet. Die Pumpe 14, das Verdampfungsgefäß 15 und das Ventilatorgehäuse 20 sind mit Schrauben oder dgl. Befestigungsmittel an einer Leiste 21 befestigt, die sich an der Innenwand des Gehäuses 1 von oben nach unten erstreckt.

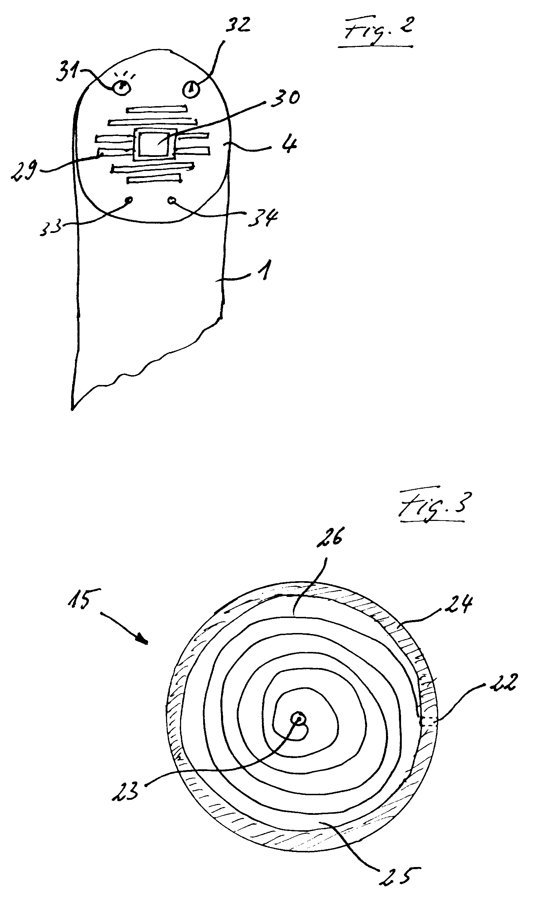

Im Betrieb pumpt die Pumpe 14 die Duftstofflösung über die Schlauchleitung 13 nach oben zum Verdampfungsgefäß 15, welches als nach oben offene Schale ausgebildet ist. Die Eintrittsöffnung 22 für die Duftstofflösung in das Verdampfungsgefäß 15 ist gemäß Fig. 3 in der Seitenwand 24 des Gefäßes 15 vorgesehen, während die Austrittsöffnung 23, an der die Rückflußleitung 16 angeschlossen ist, in der Mitte des Bodens 25 des Verdampfungsgefäßes 15 angeordnet ist. Der Boden 25 ist eben ausgebildet und mit einer Nut 26 versehen, die sich spiralförmig vom Umfang des Bodens nach innen zu der Austrittsöffnung 23 erstreckt.

Das Verdampfungsgefäß 15 besteht aus Metall, beispielsweise Aluminium, und wird durch eine elektrische Widerstandsheizung beheizt, die durch die Leitungen 27 mit Strom versorgt wird, welche an die Platine 19 angeschlossen sind. Mit der Heizeinrichtung wird der Gefäßboden 25 beispielsweise auf eine konstante Temperatur im Bereich zwischen 85 und 95°C erwärmt. Dadurch wird die mit der Pumpe 14 zugeführte Duftstofflösung, die einen Film auf dem Boden 25 des Gefäßes 15 bildet, zumindest teilweise verdampft. Der nicht verdampfte Teil fließt direkt oder über die Nut 26 langsam zu der Austrittsöffnung 23 und von dort aufgrund der Schwerkraft über die Rücklaufleitung 16 zurück zum Vorratsbehälter 9.

Der Ventilator in dem Ventilatorgehäuse 20 saugt über die in der Bodenplatte 2 vorgesehene, in der Zeichnung gestrichelt dargestellte Ansaugöffnung 28 Luft in das Gehäuse 1, die oben aus der Platte 4 durch die schlitzförmigen Luftaustrittsöffnungen 29 in den zu beduftenden Raum geblasen wird. Durch den Luftstrom in dem Gehäuse 1 wird die im Gefäß 15 verdampfte Duftstofflösung mitgerissen.

Gemäß Fig. 2 ist die Platte 4 auf dem Gehäuse 1 mit der LED-Anzeige 30 einer Schaltuhr versehen. Mit einem Drehknopf 31 auf der Platte 4 ist die Vorrichtung ein- oder ausschaltbar oder auf automatischen Betrieb gemäß der Zeitschaltuhr einschaltbar. Mit dem Drehknopf 32 auf der Platte 4 kann die Leistung des Ventilators eingestellt werden. Ein Lämpchen 33 zeigt an, wenn der Füllstand im Vorratsbehälter 9, der mit der an den Anschluß 18 angeschlossenen Sonde gemessen wird, ein vorgegebenes Niveau unterschreitet. Das weitere Lämpchen 34 zeigt den Betrieb der Vorrichtung an.

Die Schlauchpumpe 14 führt die Duftstofflösung dem Verdampfungsgefäß 15 in vorgegebenen Zeitintervallen tropfenförmig zu. Die Größe dieser Intervalle wird mit einem Schalter 35 eingestellt, der an einer Platte 36 angeordnet ist, welche an der Leiste 21 über Schraubbolzen 37 befestigt ist, die in entsprechende Bohrungen 38 eingreifen. Die Schalterbefestigungsplatte 36 weist einen weiteren Druckschalter 39 auf, der dazu dient, die Schlauchpumpe 14 zu Beginn des Betriebs einzuschalten, also die Schlauchleitung 13 bis zum Verdampfungsgefäß 15 zu füllen. Ein weiterer Schalter 40 kann zum Dauerbetrieb der Schlauchpumpe 14 vorgesehen sein, wenn die Leitungen 13 und 16 mit einer Reinigungsflüssigkeit gespült werden sollen. Die Schalter 35, 39 und 40 können auch an der Platte 4 angebracht werden. Die erfindungsgemäße Vorrichtung ist mit einem elektrischen Kabel 41 an das Stromnetz anschließbar.

Bei der erfindungsgemäßen Vorrichtung fließt also die nicht verdampfte Duftstofflösung durch die Schwerkraft über die Rücklaufleitung 16 von dem Verdampfungsgefäß 15 in den darunter angeordneten Vorratsbehälter 9 zurück. Das säulenförmige Gehäuse 1 ist mit einem Standfuß 2 versehen. Während das Gehäuse 1 oberhalb des Ventilators 20 wenigstens eine Luftaustrittsöffnung 29 aufweist, ist das Gehäuse 1 unter dem Vorratsbehälter 9 mit wenigstens einer Luftaustrittsöffnung 28 versehen. An der Oberseite des Gehäuses 1 sind die Bedienungseinrichtungen und/oder Überwachungsanzeigen angeordnet, wobei die Oberseite des Gehäuses 1 als schräg verlaufende Platte 4 ausgebildet ist. Die Rückseite des Gehäuses 1 ist als abnehmbare Abdeckung 5 ausgebildet.

Das Verdampfungsgefäß 15 ist als oben offene Schale ausgebildet. Dabei ist die Eintrittsöffnung 22 für die Duftstofflösung in das Verdampfungsgefäß 15 in der Seitenwand 24 und die Austrittsöffnung 23 für die nicht verdampfte Duftstofflösung im mittleren Bereich des Bodens 25 des Verdampfungsgefäßes 15 vorgesehen.

## Patentansprüche

1. Vorrichtung zur Abgabe von Duftstoffen in einen Raum durch Erwärmung des Duftstoffs in einem mit einer elektrischen Heizeinrichtung erwärmbaren Verdampfungsgefäß, gekennzeichnet durch eine Dosiereinrichtung, mit der eine Lösung des Duftstoffs von einem Vorratsbehälter (9) dem Verdampfungsgefäß (15) zugeführt wird.

2. Vorrichtung nach Anspruch 1, dadurch gekennzeichnet, daß der Vorratsbehälter (9) unter dem Verdampfungsgefäß (15) angeordnet ist, und die Dosiereinrichtung durch eine Pumpe (14) gebildet wird, die die Duftstofflösung von dem Vorratsbehälter (9) in das Verdampfungsgerät (15) pumpt.

3. Vorrichtung nach Anspruch 2, dadurch gekennzeichnet, daß die Pumpe (14) durch eine Schlauchpumpe gebildet wird.

4. Vorrichtung nach Anspruch 1, dadurch gekennzeichnet, daß die im Verdampfungsgefäß (15) nicht verdampfte Duftstofflösung über eine Rücklaufleitung (16) in den Vorratsbehälter (9) zurückfließt.

5. Vorrichtung nach einem der vorstehenden Ansprüche, dadurch gekennzeichnet, daß über dem Verdampfungsgefäß (15) ein Ventilator (20) angeordnet ist.

6. Vorrichtung nach einem der vorstehenden Ansprüche, dadurch gekennzeichnet, daß der Vorratsbehälter (9), das Verdampfungsgefäß (15) und der Ventilator (20) übereinander in einem als Säule ausgebildeten Gehäuse (1) ausgebildet sind.

7. Vorrichtung nach Anspruch 6, dadurch gekennzeichnet, daß das Gehäuse (1) oberhalb des Ventilators (20) wenigstens eine Luftaustrittsöffnung (29) aufweist.

8. Vorrichtung nach einem der vorstehenden Ansprüche, dadurch gekennzeichnet, daß die Eintrittsöffnung (22) für die Duftstofflösung in das Verdampfungsgefäß (5) in der Seitenwand (24) und die Austrittsöffnung (23) für die nicht verdampfte Duftstofflösung im mittleren Bereich des Bodens (25) des schalenförmigen Verdampfungsgefäßes (15) vorgesehen sind.

9. Vorrichtung nach einem der vorstehenden Ansprüche, dadurch gekennzeichnet, daß der Boden (25) des Verdampfungsgefäßes (15) eben ausgebildet ist.

10. Vorrichtung nach Anspruch 8 und 9, dadurch gekennzeichnet, daß der Boden (25) des Verdampfungsgefäßes (15) eine spiralförmige Nut (26) aufweist, die in die Austrittsöffnung (23) mündet.

11. Vorrichtung nach einem der vorstehenden Ansprüche, dadurch gekennzeichnet, daß die Duftstofflösung aus einer alkoholischen Lösung eines ätherischen Öls gebildet wird.
